# EUROPEAN PATENT SPECIFICATION

(11) **EP 1 904 493 B1**
(45) Date of publication and mention of the grant of the patent: **27.03.2013**
(21) Application number: 06753117.8
(22) Date of filing: 07.07.2006
(51) Int. Cl.: C07D 455/03, A61K 31/4375, A61P 25/00, A61P 25/06, A61P 25/16, A61P 25/18

(54) **TETRAHYDROPROTOBERBERINE COMPOUNDS, THE SYNTHETIC METHOD AND THE USE THEREOF**
TETRAHYDROPROTOBERBERINVERBINDUNGEN, SYNTHETISCHES VERFAHREN UND ANWENDUNG DAVON
COMPOSÉS TÉTRAHYDROPROTOBERBÉRINES, PROCÉDÉ DE SYNTHÈSE ET UTILISATION DE CEUX-CI

(30) Priority: 08.07.2005 CN 200510027630
(43) Date of publication of application: 02.04.2008
(73) Proprietor: Shangai Institute of Materia Medica, Chinese Academy of Sciences, Shangai (CN); Topharman Shanghai Co., Ltd., Shanghai 201209 (CN)
(72) Inventor: LI, Jianfeng, Pudong New District, Shanghai 201203 (CN); LIU, Aixiang, Pudong New District, Shanghai 201203 (CN); CHEN, Xinjian, Pudong New District, Shanghai 201203 (CN); JIN, Guozhang, Pudong New District, Shanghai 201203 (CN); YAN, Tiema, Pudong New District,2Shanghai 201203 (CN); ZHANG, Rongxia, Pudong New District, Shanghai 201203 (CN); ZHU, Yi, Pudong New District, Shanghai 201203 (CN); PAN, Yanjun, Pudong New District, Shanghai 201203 (CN); SHEN, Jingshan, Pudong New District, Shanghai 201203 (CN); SHEN, Jingkang, Pudong New District 3, Shanghai 201203 (CN)
(74) Representative: Grünecker, Kinkeldey, Stockmair & Schwanhäusser
(86) International application number: PCT/CN2006/001601
(87) International publication number: WO 2007/006212

(56) References cited:
- WO-A-2004/069144
- CN-A- 1 115 318
- CN-A- 1 603 324
- US-A- 3 932 384
- US-A- 5 470 852
- SCHAPER K.-J.: 'Free-wilson-type analysis of non-additive substituent effects on THPB' QUANTITATIVE STRUCTURE-ACTIVITY RELATIONSHIPS vol. 18, no. 4, 1999, pages 354 - 360, XP008123720
- GUO X. ET AL.: 'Characteristics of tetrahydroprotoberberines on dopamine D1 and D2 receptors in calf striatum' ZHONGGUO YAOLI XUEBAO vol. 18, no. 3, 1997, pages 225 - 230, XP008123727
- CHEN L.-J. ET AL.: 'Effect of (plus or minus) 12-chloroscoulerine on brain dopamine receptors' ZHONGGUO YAOLI XUEBAO vol. 17, no. 2, 1996, pages 185 - 189, XP008123728
- LEE S.S. ET AL.: 'Semisynthesis of tetrahydropalmatine, an active ingredients from Corydalis' COLLECTION OF CZECHOSLOVAK CHEMICAL COMMUNICATIONS vol. 55, no. 8, 1990, pages 2095 - 2099
- HANAOKA M. ET AL.: 'Chemical transformation of protoberberines. Part 10. A novel synthesis of sanguilutine and dihydrosanguilutine...' JOURNAL OF THE CHEMICAL SOCIETY, PERKIN TRANSACTIONS I: ORGANIC AND BIO-ORGANIC CHEMISTRY (1972-1999) no. 3, 1987, pages 677 - 681, XP008123730
- MCMURTREY K.D. ET AL.: 'Kinetics and product distribution in Pictet-Spengler Cyclization of tetrahydropapaveroline to tetrahydroprotoberberine alkaloids' JOURNAL OF ORGANIC CHEMISTRY vol. 49, no. 5, 1984, pages 947 - 948, XP003007529
- LIN Z. ET AL.: 'Structural elucidation and total synthesis of Corydalis L' FUDAN XUEBAO, ZIRAN KEXUEBAN vol. 20, no. 4, 1981, pages 446 - 449, XP008123731
- PANDEY G.D. ET AL.: 'Synthesis of heterocycles via lactones. Part III. A synthesis of berbines' HETEROCYCLES vol. 12, no. 10, 1979, pages 1327 - 1330, XP008123732
- O'BRIEN J.P. ET AL.: 'Preferential cleavage of the methoxyl group adjacent to a phenolic function in polymethoxylated isoquinolines' HETEROCYCLES vol. 11, 1978, pages 347 - 350, XP008123734
- DATABASE CAPLUS [Online] CHIANG H.-C. ET AL.: 'Synthesis of (plus or minus)-govadine', XP003007866 Database accession no. (1979:405394) & TAIWAN YAOXUE ZAZHI vol. 30, no. 1, 1978, pages 54 - 62
- SHEPPARD H. ET AL.: 'The dopamine-sensitive adenylate cyclase of the rat caudate nucleus. 3' BIOCHEMICAL PHARMACOLOGY vol. 27, no. 8, 1978, pages 1113 - 1116, XP003002656
- TEITEL S. ET AL.: 'Preferential removal of a methylenedioxy group from optically active isoquinolines' HETEROCYCLES vol. 5, no. 1, 1976, pages 85 - 90, XP008123735
- KAMETANI T. ET AL.: 'Synthesis of heterocyclic compounds. CDXXI...' JOURNAL OF THE CHEMICAL SOCIETY USECTION] C: ORGANIC no. 20, 1971, pages 3318 - 3321, XP008123736
- TANI C. ET AL.: 'Studies on the Alkaloids of fumariaceos plants. X. Alkaloids of Corydalis platycarpa' YAKUGAKU ZASSHI vol. 90, no. 3, 1970, pages 407 - 411, XP008123723
- SUN T.-C. ET AL.: 'Synthesis of compounds related to corydalis B (tetrahydropalmatine). IV' YAOXUE XUEBAO vol. 12, no. 5, 1965, pages 314 - 318, XP001539885
- TOMITA M. ET AL.: 'The alkaloids of menispermaceous plants. CCVI. Alkaloids of formosan Stephania japonica. 3' YAKUGAKU ZASSHI vol. 84, no. 8, 1964, pages 776 - 778, XP008123737
- TUNG-LU P. ET AL.: 'Synthesis of tetrahydropalmatine analogs. I. Esterification and alkylation...' SCIENTIA SINICA (ENGLISH EDITION) vol. 12, no. 2, 1963, pages 191 - 199, XP008123738
- TOMITA M. ET AL.: 'Reaction of tetrahydropalmatine and tetrahydrojatrorrhizine with metallic sodium in liquid ammonia' YAKUGAKU ZASSHI vol. 79, 1959, pages 690 - 692, XP008123739
- SPATH E. ET AL.: 'Alkaloids of colombo root. V. A new base of colombo root and the constitution of berberrubine and palmatrubine' BER. vol. 59B, 1926, pages 1486 - 1496, XP008124708

## Description

### Technical field

The present invention relates to novel tetrahydroprotoberberines. The compounds possess valuable therapeutic properties and are suitable, especially, for treating diseases that respond to modulation of dopamine receptors, such as schizophrenia, parkinsonism, hyperactivity disorder, migraine etc.

### Background art

Recently, the pathogenesis of schizophrenia has been suggested to involve dysfunction of dopamine D₁ receptors in the medial prefrontal cortex (mPFC), which is resulted in dopamine D₂ receptor hyperactivity in subcortical regions such as the ventral tegmental area (VTA) and the nucleus accumbens (NAc). D₁ receptors dysfunction is involved in the negative symptoms of schizophrenia whereas the D₂ receptors hyperactivity results in the positive symptoms of this disorder. According to this new hypothesis, an effective antipsychotic drug should have both D₁ receptor agonist and D₂ receptor antagonist dual actions.

Tetrahydroprotoberberine analogues (THPBs) have this dual actions, including *l*-Stepholidine (*I*-SPD) and *l*-chloroscoulerine (ZL94112235.2, CN03151464.2). *l*-SPD is an active ingredient of the Chinese herb *Stephania. l*-CSL is a derivative of *l*-SPD. In preliminary clinic studies, *l*-SPD showed favorable activity and few side effects in the treatment of schizophrenia. So, the efficacy of THPBs for neuron system disease especilly schizophrenia merits further investigation.

WO 2004/069144 relates to the use of tetrahydroprotoberberine, tetrahydropalmatine and stepholidine for use in decreasing nicotine use.

CN 1115318 describes tetrahydroberberines with strong medicinal activity.

US 5,470,852 discloses the use of tetrahydroprotoberberine compounds for treating for treating arrhythmia.

Sheppard and Brughardt, Biochemical Pharmacology, Vol 27, No. 8, 1978, pages 1113 to 1116 tested the activity of a series of aporphines and protoberberines on adenylate cyclases

### Disclosure Of The Invention

The invention is based on the object of providing compounds of THPBs, as well as its pharmacologically acceptable salts and solvates, which act as highly affinity dopamine receptor ligands.

The present invention also relates to a preparation method of compounds of THPBs.

The present invention also relates to a method for treating disorder which respond to influencing by dopamine D₁ and D₂ receptor. And a method comprise administering an effective amount of at least one THPBs of the formula (I) and/or at least one physiologically acceptable salt of formula(I) to a subject in need thereof.

The present invention relates to the compounds of formula (I), pharmacologically acceptable salts and solvates: 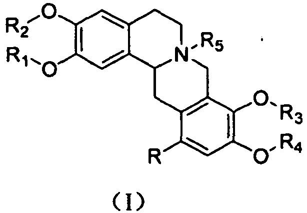 Wherein
R is H;
R₁ is H, COR₇, or R₁ and R₂ together form CH₂; R₇ is selected from C₁-C₁₂ alkyl, COR₈, alkoxy, alkyl substituted by (CH₂CH₂O)ₙR₆ (n=1~3); R₆ is selected from H, C₁-C₃ alkyl or alkyl substituted by aryl; R₈ is selected from alkoxy;
R₂ is H, methyl, or R₁ and R₂ together form CH₂;
R₃ is H, methyl, or COR₇;
R₄ is H, methyl, or COR₇;
R₅ is H, O, C₁∼C₃ alkyl, substituted C₁∼C₃ alkyl, or aryl, or there is no existing of R₅,
among the above compounds,
When R₁ is H and R₂=R₃=CH₃, R₄ is COR₇.
When R₁ is H and R₂=R₄=CH₃, R₃ is COR₇.
When R₁ and R₂ together form CH₂, R₃ or R₄ can't be selected from H, CH₃, or COCH₃ at the same time,
at least one of R₁, R₃ or R₄ is an acyl group;
the said provisos exclude the following known compounds:
(1) 3,9,10-trimethoxy-2-acetoxy-5,8,13,13a-tetrahydro-6*H*-dibenzo[a, g]quinolizine;
(2)2,3-methylenedioxyl-9-*p*-methoxybenzoyloxy-10-methoxy-5,8,13,13a-tetrahy dro-6*H*-dibenzo[a, g]quinolizine;
(3)2,3-methylenedioxyl-9-(3,4,5-trimethoxybenzoyloxy)-10-methoxy-5,8,13,13a -tetrahydro-6*H*-dibenzo[a, g]quinolizine.

Formula (I) has one or several chiral carbons. So the chiral isomers exist, including enantiomers, unenantiomers or its mixture. This invention including the R-, and S- enantiomers and its mixture. The enantiomer can be separated by optical resolution with chemical method or separated by chiral HPLC. It also can be obtained by asymmetry synthesis.

The present invention relates to radioactivity derivatives of formula (I), which is suitable for biological studies.

The present invention also relates to the physiologically acceptable acid addition salts and alkali derivatives of formula (I). The acid addition salts are salts of the compounds of formula (I) with acid, including hydrochloric acid, hydrobromic acid, sulfuric acid, phosphoric acid, cabonic acid, organic sulfonic acid. The alkali derivatives is salts obtained by reacting the compounds of formula (I) with alkali, especially alkali metal derivatives, including natrium or potassium derivatives.

The following compounds of formula (I) are particularly preferred:
2,9-diacetoxy-3,10-dimethoxy-5,8,13,13a-tetrahydro-6*H*-dibenzo[a, g] quinolizine;
(-)-2,10-diacetoxy-3,9-dimethoxy-5,8,13,13a-tetrahydro-6*H*-dibenzo[a, g] quinolizine;
(-)-2,10-divalyloxy-3,9-dimethoxy-5,8,13,13a-tetrahydro-6*H*-dibenzo[a, g]quinolizine.

The present invention also related to the method of preparing the compounds of formula (I) and its derivatives.

The compounds of the formula (I) can be prepared by analogy to methods which are well known in the literatures. A preferred method for the preparation of compounds (I) is outlined below:

### 1. Preparation from formula (II):

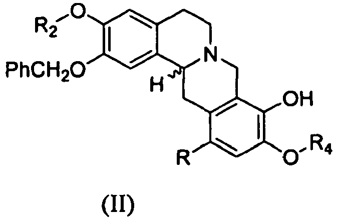

Compound of formula (II) is reacted with compound of formula (III-b) to prepare compound of formula (I):

R₃Y (III-b)

Wherein R₃ is defined as described above, Y is halogen or hydroxy.
(1) Compound of formula (II) is reacted with R₃Cl or R₃Br. The temperature is in the range of 0 °C -100 °C. The reaction is catalysted by appropriate alkali. In detail, in the present of inorganic alkali (for example: NaOH, KOH, CsOH, Ba(OH)₂, Mg(OH)₂, Ca(OH)₂, KHCO₃, K₂CO₃, Na₂CO₃, Cs₂CO₃) or organic alkali (for example: sodium alkoxide, NEt₃, N(C₄H₉)₃, N(C₃H₇)₃, et al), the mixture was stirred at 0-100 °C for 2-24 hours to give compound of formula (I). The solvent can be selected in alcohol (for example: methanol, ethanol, isopropanol, C₄H₉OH, iso-C₄H₉OH, t-C₄H₉OH, C₅H₁₁OH, iso-C₅H₁₁OH), the mixture solution of alcohol and water (alcohol: water=5: 9.5-9.5: 0.5, V: V) or other solvent (for example: DMF, CH₂Cl₂, DMSO, THF, dioxane, pyrolidinylone, acetone and CH₃OCH₂CH₂OCH₃).
(2) Compound of formula (II) is treated with R₃COCl, R₃CO)₂O or R₃CO)₂O. The reaction carries through in the present of appropriate alkali at 0-100 °C. In detail, in the present of inorganic alkali (for example: NaOH, KOH, CsOH, Ba(OH)₂, Mg(OH)₂, Ca(OH)₂, KHCO₃, K₂CO₃, Na₂CO₃, Cs₂CO₃) or organic alkali (for example: pyridine, NEt₃, N(C₄H₉)₃, N(C₃H₇)₃, et al), the mixture was stirred at 0-100 °C for 2-8 hours to give compound of formula (I). The solvent can be selected in pyridine, DMF, CH₂Cl₂, DMSO, THF, dioxane and pyrrolidone derivatives. The catalyst such as DMAP is added according the reaction conditions.
(3) When R₁=H, compound of formula (I) was obtained by hydrogenating the compound of formula (II) (the compound of formula (I) with R₁=CH₂Ph) in the present of catalyst in order to avoid the hydrogenation of halogen in THPBs. In detail, compound of formula (II) is hydrogenated in the present of Raney-Ni at 0-40 °C for 1-10 hours to give compound of formula (I). The solvent is alcohol (for example: methanol, ethanol, isopropanol, et al) or the mixture solution of alcohol and water.

Compound of formula (II) is given as follow:
A) When R₂=R₄=CH₃ and R = Cl, that is 2-benzyloxy-3,10-dimethoxy-9-hydroxy-12-chloro-5,8,13,13a-tetrahydro-6*H*-dibenzo[a, g]quinolizine (compound IIA). Intermediate IIA is prepared according to the method reported in literature (CN03151464.2), and (-)-IIA is obtained through resolution from IIA (CN03151464.2).
B) When R₂, R₄ is other substituted groups, the hydroxyl derivatives of THPBs can be obtained by debenzyloxy or demethyl reaction of compound of formula (II) (R₂=R₄=CH₃) in the present of BBr₃. This compound is esterificated, etherificated, coupled with amino acid or demethylatied to provide the compound of formula (II). The procedure is described above.

### 2. Preparation from formula (IV):

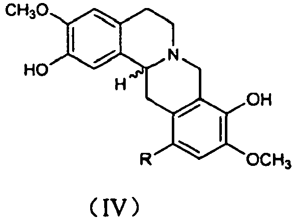

R is H.

Compound of formula (IV) is treated with compound of formula (III-a) or (III-b) to prepare the compound of formula (I):

R₁ Y (III-a) or R₃Y (III-b)

Wherein R₁ (or R₃) is definited as described above, Y is halogen or hydroxy.
(1) Compound of formula (IV) is reacted with R₁Cl or R₁Br. The procedure is the same as the synthetic procedure described in the reaction of compound of formula (II) with R₃Cl or R₃Br. The structure of compound of formula (I) is different according the amount of compound of formula (III-a) or (III-b) and the activity of the hydroxy of compound of formula (IV).
(2) Compound of formula (IV) is reacted with R₁COCl, (R₁CO)₂O or (R₃CO)₂O. The procedure is the same as the synthetic procedure described in the reaction of compound of formula (II) with R₁COCl, (R₁CO)₂O or (R₃CO)₂O. The structure of compound of formula (I) is different according the amount of compound of formula (III-a) or (III-b) and the activity of the hydroxy of compound of formula (IV).

Compound of formula (IV) is given as follow:

Compound of formula (II) (R₂=R₄=CH₃, R=Cl) is hydrogenated in the present of Pd-C under high pressure to give formula (IV) (R=H). In detail, compound of formula (II) is hydrogenated at 20-60 °C for 3∼24 hours in the present of acid (for example: HCl/H₂O, H₂SO₄/H₂O, HBr/H₂O, et al) to give compound of formula (IV) (R=H). The solvent is alcohol (for example: methanol, ethanol, isopropanol, et al) or other solvent (DMF, DMSO, THF, et al).

### 3. Preparation from formula (V):

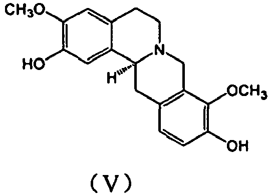

Compound of formula (V) is natural product *l*-SPD, isolated from plants. Staring from *l*-SPD, the compound of formula (I) can be provided by esterification, etherification, coupling with amino acid or demethylation. The procedure is described above.

### 4. Preparation from formula (VI):

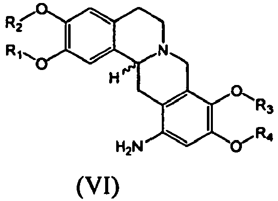

Compound of formula (VI) was reacted with HNO₂ to give diazo salts, which is reacted with corresponding reagent to give the compound of formula (I).

Compound of formula (VI) can be prepared as follow:

Nitration of compound of formula (I) (R=H) to give nitro derivatives, which is reduced to give compound of formula (VI). The nitro derivatives of compound of formula (I) also can be obtained by cyclization of nitro benzylisoqunoline derivatives.

In the formulae (II)∼(VI), the definitions for R, R₁, R₂, R₃ and R₄ are the same as those in formula (I).

The physiologically acceptable acid addition salts of formula (I) can be obtained by the conventional methods in the literature. For example, the compound of formula (I) was treated with appropriate acid in appropriate solvent, and the salt can be obtained through evaporating solvents or filtrating.

The compound of formula (I) was treated with appropriate alkali to give physiologically acceptable alkali addition salts.

The pharmacological actions of the compound according to the present invention are detected through the following methods.

### 1. Dopamine receptor binding studies

The affinities of these compounds on dopamine receptors were evaluated with competitive receptor binding assays (Acta Pharmacol Sin, 1989, 10:104 and Acta Pharmacol Sin, 2003, 24(3): 225-229). The Ki is calculated according the inhibition data tested.

*l*-CSLMS is S-chloroscoulerine mesylate and CSL is chloroscoulerine.

**Table 1 Affinities of THPBs for binding to dopamine receptors^{a}**

| Example | Inhibition(%) for D₁ | Inhibition(%) for D₂ |
|---|---|---|
| **2** | 99.4 | 90.7 |

| | | |
|---|---|---|
| a : Inhibition(%) data was tested at 1 × 10⁻⁵mol/L | | |

**Table 2 Ki values of THPBs for bingding to dopamine receptors**

| Example | Ki | |
|---|---|---|
| | D₁(nM) | D₂(µM) |
| *l*-CSLMS | - | 1.7±0.5 |
| CSL | 6.1±3.0 | - |
| **2** | 51.8±32.2 | 10.6±2.6 |

### 2. 6-OHDA-lesioned rats rotation test

Some of the compounds were evaluated on 6-OHDA-lesioned rats model. In this model, compound of example 3 exhibit positive effects at 10mg/kg.

The present invention therefore relates to compounds of formula (I) and physiologically acceptable salts.

The present invention therefore relates to a pharmaceutical composition which comprises at least one compound of the formula (I) and/or at least one physiologically acceptable addition salt of (I), together with physiologically acceptable carriers and/or auxiliary substances.

The present invention also relates to a method for treating disorder which respond to influencing by dopamine D₁ and D₂ receptor. And a method comprise administering an effective amount of at least one THPBs of the formula (I) and/or at least one physiologically acceptable addition salt of (I) to a subject in need thereof.

The present invention also relates to all the new intermediates described in this invention.

### The Preferable Embodiments of the Invention

The following examples serve to explain the invention.

The compounds were characterized either via ¹H NMR or MS.

The compound in formula(II), when R₂=R₄=CH₃, R=Cl, i.e. the intermediate 2-benzyloxy-3, 10-dimethoxy-9-hydroxy-12-chloro-5,8,13,13a-tetrahydro-6H-dibenzo [a, g]quinolizine (compound IIA), which is prepared according to the method reported in literature (CN03151464.2), (-)-IIA is obtained through the resolution from IIA (CN03151464.2).

### Preparation 1: 2-benaloxy-3,10-dimethoxy-9-hydroxy-12-chloro-5,8,13,13a-tetrahydro-6H-dibenzo[a,g] quinolizine(compound IIA)

1-(2'-chloro-4'-methoxy-5'-hydroxy)benzyl-6-methoxy-7-benzyloxy-1,2,3,4-tetro hydroisoquinolin (compound of formula VII) (103g, 0.235mol) was added methanol (5150ml) and hydrochloric acid to adjust pH1-2. The mixture was then added 37%HCHO (3090ml) and water (2000ml). After stirring for 2 days, the solvent was evaporated and the residue was neutralized with NaHCO₃ to pH 9. The aqueous was extract with CHCl₃. The combined organic layers was dried and concentrated to give desired compound (103g, 97.3%).

### Preparation 2: 2,9-dihydroxy-3,10-dimethoxy-5,8,13,13a-tetrahydro-6H-dibenzo [a, g]quinolizine hydrochloride

Compound IIA (2.0g, 4.4mmol) was dissolved in CH₃OH (200ml), adding 1mol/L hydrochloric acid (5ml) and 10% Pd-C (0.3g). The mixture was hydrogenated under pressure at 50∼60 °C for 12 hours. After the raw material disappeared detected with TLC, filtering the catalyst, the filtrate was concentrated to give the product as beige powder (1.5g, 93%). That is scoulerine, which can be used as a intermediate for next step. mp 246 °C. ¹HNMR (DMSO-d₆)δ: 2.46∼2.60 (3H, m, CH₂), 3.13 (1H, m, CH₂), 3.14∼3.30 (4H, m, CH₂ or N-CH), 3.74 (3H, s, Ar-OCH₃), 3.76 (3H, s, Ar-OCH₃), 4.03 (1H, d, CH₂), 6.59 (1H, d, ArH), 6.64 (1 H, s, ArH), 6.70 (1H, s, ArH), 6.78 (1H, d, ArH).

### Reference Example 1: 2-benzyloxy-3,10-dimethoxy-9-(N-t-Boc-phenylalanyloxy)-12-chloro-5,8,13,13a-tetrahydro-6H-dibenzo[a, g]quinolizine

### Method A:

Compound IIA (0.5g, 1.1 mmol) was dissolved in CH₂Cl₂ (20ml), adding t-Boc-phenylanine (0.88g, 3.3mmol) and DCC (0.91 g, 4.4mmol). The mixture was stirred at room temperature for 6 hours, then cooled in refrigerator. After filtered, the filtrate was washed with cool citrate buffer, cool saturated NaHCO₃ and cool water. The organic layer was dried over Na₂SO₄, filtered, and the solvent was evaporated under reduced pressure. The crude product was purified by silica chromatography to give pale yellow solid (0.722g, 93.3%). mp 140∼141 °C. ¹HNMR (CDCl₃)δ: 1.41 (9H, 2×s, 3×CH₃), 2.46∼2.68 (3H, m, CH₂), 3.03∼3.19 (4H, m, CH₂), 3.28∼3.49 (3H, m, CH₂ and N-CH), 3.79 (3H, d, Ar-OCH₃), 3.88(3H, s, Ar-OCH₃), 4.85 (1H, m, CH₂), 4.98 (1H, m, COCH), 5.17 (2H, q, PhCH₂), 6.63 (1H, s, ArH), 6.76 (1H, s, ArH), 6.90 (1H, s, ArH), 7.28∼7.40 (8H, m, PhH), 7.46∼7.48 (2H, m, PhH). MS(EI) m/z: 698 (M⁺), 641, 450, 434(base), 360, 91.

### Method B:

t-Boc-phenylanine (0.177g, 0.67mmol) and CDI (0.227g, 1.4mmol) was dissolved in dry THF (10ml) and stirred at room temperature for 30 minutes. Then the solution of compound IIA (0.3g, 0.66mmol) in THF (10ml) was added and stirred for one day. The mixture was evaporated under reduced pressure and purified by Al₂O₃ chromatography to give beige solid (0.114g, 24.6%).

### Reference Example 2: 2-benzyloxy-3,10-dimethoxy-9-phenylalanyloxy-12-chloro-5,8, 13,13a-tetrahydro-6H-dibenzo[a, g]quinolizine

A product obtained in Reference Example 1 (0.209g, 0.30mmol) was stirred with 10%CF₃COOH in CH₂Cl₂ at room temperature for 2 hours. Then the mixture was concentrated and added water, adjusting to pH 8 with saturated NaHCO₃. The aqueous phase was extracted with CH₂Cl₂. The combined organic layers were washed with brine and dried over Na₂SO₄, filtered, and the solvent was evaporated under reduced pressure. The residue was purified by silica chromatography to give desired product (0.026g, 14.5%). ¹HNMR (CDCl₃)δ: 2.46∼2.69 (3H, m, CH₂), 2.96∼3.49 (7H, m, CH₂ and N-CH), 3.79 (3H, d, Ar-OCH₃), 3.85 (1H, s, CH₂), 3.88(3H, s, Ar-OCH₃), 4.03∼4.08(1H, m, COCH), 5.17 (2H, q, PhCH₂), 6.64 (1H, s, ArH), 6.77 (1H, s, ArH), 6.91 (1H, s, ArH), 7.28∼7.48 (10H, m, PhH).

### Preparation 3: (-)-2,10-bis-(N-t-Boc-valyloxy)-3,9-dimethoxy-5,8,13,13a-tetrahydro-6H-dibenzo[a, g]quinolizine

*l*-SPD (0.09g, 0.27mmol), t-Boc-valine (0.144g, 0.66mmol), DCC (0.189g, 0.92mmol), DMAP (0.049g, 0.4mmol) and CH₂Cl₂ (10ml) was mixed and stirred at room temperature for 48 hours. The mixture was then cooled in refrigerator. After filtered, the filtrate was washed with cool citrate buffer, cool saturated NaHCO₃ and brine. The organic layer was dried over Na₂SO₄, filtered, and the solvent was evaporated under reduced pressure. The crude product was purified by silica chromatography (ethyl acetate/petroleum ether) to give pale yellow solid (0.115g, 57.6%). ¹HNMR (CDCl₃)δ: 1.02∼1.03 (6H, d, 2×CH₃), 1.08∼1.11 (6H, q, 2×CH₃), 1.46∼1.48 (18H, d, 6×CH₃), 2.36∼2.42 (2H, m, CH₂), 2.63∼2.75 (2H, m, CH₂), 2.83∼2.89 (1H, m, CH₂), 3.14∼3.24 (3H, m, CH₂ and CH), 3.53∼3.57 (2H, m, CH₂ and N-CH), 3.78 (3H, s, Ar-OCH₃), 3.79(3H, s, Ar-OCH₃), 4.18 (1H, d, CH₂), 4.52 (1H, d, COCH), 4.54 (1H, d, COCH), 6.70 (1H, s, ArH), 6.91 (3 H, m, ArH).

### Example 1: (-)-2,10-divalyloxy-3,9-dimethoxy-5,8,13,13a-tetrahydro-6H-dibenzo[a, g]quinolizine

A product obtained in preparation 3 (0.1g, 0.14mmol) was treat with 10% CF₃COOH in CH₂Cl₂. The desired product was obtained following the synthetic procedure described in Reference Example 2.

### Example 2: 2,9-diacetoxy-3,10-dimethoxy-5,8,13,13a-tetrahydro-6H-dibenzo[a, p]quinolizine

A product obtained in preparation 2 (0.1g, 0.27ml) was dissolved in pyridine (10ml), adding acetyl anhydride (0.5ml). The mixture was stirred at room temperature for 2 hours. The mixture was concentrated under reduced pressure and added water. Then the aqueous phase was adjust to pH 8 with saturated NaHCO₃ and extracted with CH₂Cl₂. The combined organic layers were washed with brine and dried over Na₂SO₄, filtered, and the solvent was evaporated under reduced pressure to give pale desired product (0.073g, 64.6%). mp 212∼214 °C. ¹HNMR (CDCl₃)δ: 2.33 (3H, s, COCH₃), 2.34 (3H, s, COCH₃), 2.63∼2.75 (3H, m, CH₂), 3.13∼3.20(3H, m, CH₂), 3.41∼3.56 (2H, m, CH₂ and N-CH), 3.81 (3H, s, Ar-OCH₃), 3.82(3H, s, Ar-OCH₃), 4.02 (1H, d, CH₂), 6.70 (1H, s, ArH), 6.83 (1H, d, ArH), 6.92 (1H, s, ArH), 7.00 (1H, s, ArH).

### Example 3 : (-)-2,10-diacetoxy-3,9-dimethoxy-5,8,13,13a-tetrahydro-6H-dibenzo[a, g]quinolizine

*l*-SPD (0.264g, 0.81mmol), pyridine (1ml) and acetyl anhydride (0.5ml) was mixtured and stirred at room temperature for 0.5 hours. The mixture was poured into water, then yellow solid was precipitated. The solid was collected by filtering and wash with water, then dried at 40 °C to give pale yellow solid (0.235g, 70.8%). Anal:cacl:C 67.14%, H 6.12%, N 3.40%; test: C 67.46%, H 5.97%, N3.30%. ¹HNMR (CDCl₃)δ:2.32 (6H, 2s, 2×COCH₃), 2.61∼2.91 (3H, m, CH₂), 3.14∼3.29 (3H, m, CH₂), 3.51∼3.60 (2H, m, CH₂ and N-CH), 3.81 (3H, s, Ar-OCH₃), 3.82(3H, s, Ar-OCH₃), 4.20 (1H, d, J=15.9Hz, CH₂), 6.71 (1H, s, ArH), 6.90 (2H, s, ArH), 6.92 (1H, s, ArH).MS(EI) m/z:411 (M⁺), 368, 220, 176, 150(base), 135.

## Claims

1. Compounds of the formula (I), their stereoisomers, pharmacologically acceptable salts and solvates: 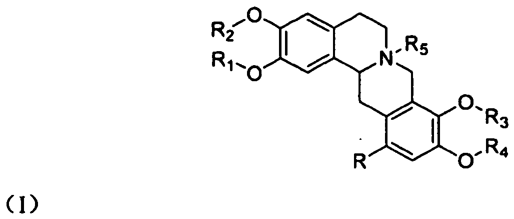 wherein
R is H;
R₁ is H, COR₇ or R₁ and R₂ together form CH₂; R₇ is selected from C₁-C₁₂ alkyl, COR₈, alkoxy, or alkyl substituted by (CH₂CH₂O)ₙR₆ (n=1∼3); R₆ is H, C₁~C₃ alkyl or alkyl substituted by aryl; R₈ is alkoxy;
R₂ is H, methyl, or R₂ and R₁ together form CH₂;
R₃ is H, methyl or COR₇;
R₄ is H, methyl or COR₇;
R₅ is H, O, C₁∼C₃ alkyl, substituted C₁∼C₃ alkyl, or aryl, or there is no existing of R₅;
Among the above compounds,
when R₁=H and R₂=R₃=CH₃, R₄ is COR₇;
when R₁=H and R₂=R₄=CH₃, R₃ is COR₇;
when R₁ and R₂ together form CH₂, R₃ and R₄ can't be selected from H, CH₃, or COCH₃ at the same time;
at least one of R₁, R₃ or R₄ is an acyl group;
the said provisos exclude the following known compounds:
(1) 3,9,10-trimethoxy-2-acetoxy-5,8,13,13a-tetrahydro-6H-dibenzo[a, g]quinolizine;
(2) 2,3-methylenedioxyl-9-p-methoxybenzoyloxy-10-methoxy-5,8,13,13a-tetrahydro-6H-dibenzo[a,g]quinolizine;
(3) 2,3-methylenedioxyl-9-(3,4,5-trimethoxybenzoyloxy)-10-methoxy-5,8,13,13a-tetrahydro-6H-dibenzo[a,g]quinolizine.

2. The compounds according to claim 1, wherein the said compounds of formula (I) are the following compounds:
2,9-diacetoxy-3,10-dimethoxy-5,8,13,13a-tetrahydro-6*H*-dibenzo[a,g]quinolizin;
(-)-2,10-diacetoxy-3,9-dimethoxy-5,8,13,13a-tetrahydro-6*H*-dibenzo[a,g]quinolizine;
(-)-2,10-divalyloxy-3,9-dimethoxy-5,8,13,13a-tetrahydro-6*H*-dibenzo[a,g]quinolizine.

3. The compounds according to claim 1, wherein the physiologically acceptable salts are salts of the compounds of formula (I) with acid, including hydrochloric acid, hydrobromic acid, sulfuric acid, phosphoric acid, cabonic acid, organic sulfonic acid; or salts of the compounds of formula (I) with alkali, especially alkali metal derivatives, including sodium or potassium derivatives.

4. The preparation method of the compounds according to claim 1:
(1) Preparation from formula (II): 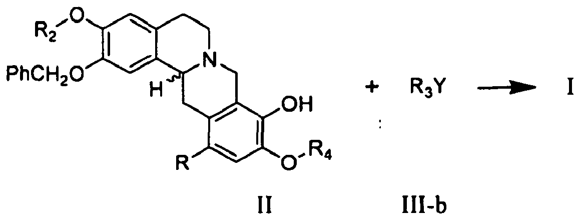 Compound of formula (II) is reacted with compound of formula (III-b) to prepare compound of formula (I):
1) Compound of formula (II) is reacted with R₃Cl or R₃Br; the temperature is in the range of 0 °C -100 °C, the reaction is catalysted by inorganic alkali or organic alkali, wherein the inorganic alkali is NaOH, KOH, CsOH, Ba(OH)₂, Mg(OH)₂, Ca(OH), KHCO₃, K₂CO₃, Na₂CO₃ or Cs₂CO₃, the organic alkali is sodium alkoxide, NEt₃, N(C₄H₉)₃ or N(C₃H₇)₃, the mixture was stirred at 0-100 °C for 2-24 hours to give compound of formula (I); the solvent is selected from the group including methanol, ethanol, isopropanol, C₄H₉OH, iso-C₄H₉OH, t-C₄H₉OH, C₅H₁₁OH, iso-C₅H₁₁OH, and the mixture of the above alcohol and water, in which alcohol: water=5: 9.5-9.5: 0.5(V: V) , or from the group including DMF, CH₂Cl₂, DMSO, THF, dioxane, pyrrolidone derivatives, acetone and CH₃OCH₂CH₂OCH₃;
2) Compound of formula (II) is reacted with R₃COCl, (R₃CO)₂O or R₃(CO)₂O; the reaction carry through in the present of inorganic alkali or organic alkali at 0-100 °C, wherein the inorganic alkali is NaOH, KOH, C₅OH, Ba(OH)₂, Mg(OH)₂, Ca(OH)₂, KHCO₃, K₂CO₃, Na₂CO₃ or Cs₂CO₃, the organic alkali is pyridine, NEt₃, N(C₄H₉)₃, N(C₃H₇)₃; the mixture is stirred at 0-100 °C for 2-8 hours to give compound of formula (I); the solvent is selected in pyridine, DMF, CH₂Cl₂, DMSO, THF, dioxane and pyrrolidone derivatives; The catalyst DMAP is added according the reaction conditions;
3) When R₁=H, compound of formula (I) is obtained by hydrogenating the compound of formula (II) (the compound of formula (I) with R₁=CH₂Ph) in the present of catalyst at 0-40 °C for 1-10h; the catalyst is Raney-Ni, the solvent is methanol, ethanol, isopropanol or the mixture solution of the above alcohol and water;
(2)Preparation from formula (IV): 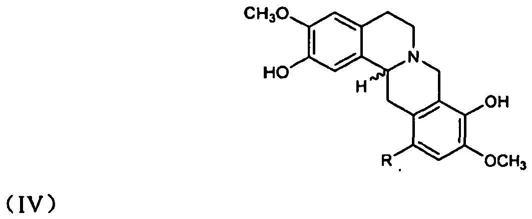 Compound of formula (IV) is reacted with compound of formula (III-a) or (III-b) to prepare the compound of formula (I):
R₁Y (III-a) or R₃Y (III-b)
1) Compound of formula (IV) is reacted with R₁Cl or R₁Br in defferent ratio of amount to give compound of formula (I); 2) Compound of formula (IV) is reacted with R₁COCl, (R₁CO)₂O or (R₃CO)₂O in different ratio of amount to give compound of formula (I);
(3) Preparation from formula (V): 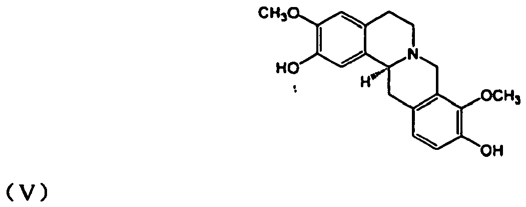 Compound of formula (V) is *l-*SPD. Staring from *l*-SPD, the compound of formula (I) is provided by esterification, etherification, coupling with amino acid or demethylation;
(4) Preparation from formula (VI): 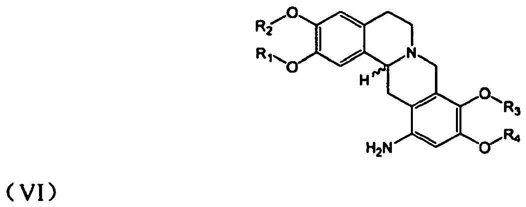
Compound of formula (VI) is treated with HNO₂ to give diazo salt, which is reacted with corresponding reagent to give the compound of formula (I),
wherein, the definitions for R, R₁, R₂, R₃ and R₄ are the same as described in Claim 1.

5. The preparation method according to claim 4, wherein the benzyloxy derivatives of compound of formula (I) is debenzylated in the present of Raney-Ni at 0-40 °C for 1-10 hours to give compound of formula (I); the solvent is alcohol, or the mixture solution of alcohol and water.

6. The use of the compound according to claim 1 in the preparation of the medicines for prevention or treatment of the disease of the central nervous system.

7. The use according to claim 6, wherein the disease of the central nervous system is schizophrenia, parkinsonism, hyperactivity disorder or migraine.

## Patentansprüche

1. Verbindungen der Formel (I), ihre Stereoisomere, pharmakologisch verträglichen Salze und Solvate: 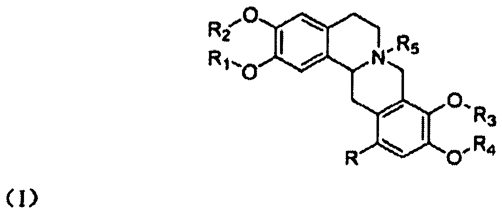 wobei
R ist H;
R₁ ist H, COR₇ oder R₁ und R₂ bilden zusammen CH₂; R₇ ist ausgewählt aus C₁-C₁₂-Alkyl, COR₈, Alkoxy, oder Alkyl substituiert durch (CH₂CH₂O)ₙR₆ (n = 1-3); R₆ ist H, C₁-C₃-Alkyl oder Alkyl substituiert durch Aryl; R₈ ist Alkoxy;
R₂ ist H, Methyl, oder R₂ und R₁ bilden zusammen CH₂;
R₃ ist H, Methyl oder COR₇;
R₄ ist H, Methyl oder COR₇;
R₅ ist H, O, C₁-C₃-Alkyl, substituiertes C₁-C₃-Alkyl, oder Aryl, oder es gibt kein R₅;
für die vorstehenden Verbindungen gilt:
wenn R₁=H und R₂=R₃=CH₃, ist R₄ COR₇;
wenn R₁=H und R₂=R₄=CH₃, ist R₃ COR₇;
wenn R₁ und R₂ zusammen CH₂ bilden, können R₃ und R₄ nicht gleichzeitig aus H, CH₃ oder COCH₃ ausgewählt sein;
wenigstens eines von R₁, R₃ oder R₄ ist eine Acylgruppe;
die besagten Maßgaben schließen die folgenden bekannten Verbindungen aus:
(1) 3,9,10-Trimethoxy-2-acetoxy-5,8,13,13a-tetrahydro-6*H*-dibenzo[a, g]chinolizin;
(2) 2,3-Methylendioxyl-9-*p*-methoxybenzoyloxy-10-methoxy-5,8,13,13a-tetrahydro-6*H-*dibenzo[a, g]chinolizin;
(3) 2,3-Methylendioxyl-9-(3,4,5-trimethoxybenzoyloxy)-10-methoxy-5,8,13,13a-tetrahydro-6*H*-dibenzo[a, g]chinolizin.

2. Verbindungen nach Anspruch 1, wobei die Verbindungen der Formel (I) die folgenden Verbindungen sind:
2,9-Diacetoxy-3,10-dimethoxy-5,8,13,13a-tetrahydro-6*H*-dibenzo[a,g]chinolizin;
(-)-2,10-Diacetoxy-3,9-dimethoxy-5,8,13,13a-tetrahydro-6*H*-dibenzo[a,g]chinolizin;
(-)-2,10-Divalyloxy-3,9-dimethoxy-5,8,13,13a-tetrahydro-6*H*-dibenzo[a,g]chinolizin.

3. Verbindungen nach Anspruch 1, wobei die physiologisch verträglichen Salze Salze der Verbindungen der Formel (I) mit Säure, einschließlich Chlorwasserstoffsäure, Bromwasserstoffsäure, Schwefelsäure, Phosphorsäure, Kohlensäure, organischer Sulfonsäure; oder Salze der Verbindungen der Formel (I) mit Alkali, insbesondere Alkalimetallderivaten, einschließlich Natrium- oder Kaliumderivaten, sind.

4. Herstellungsverfahren für die Verbindungen nach Anspruch 1:
(1) Herstellung aus Formel (II): 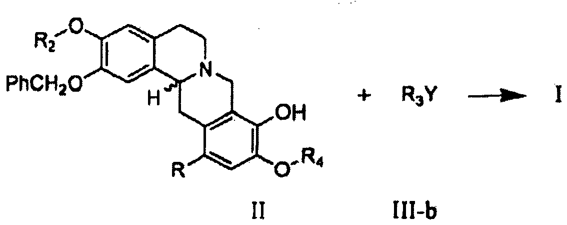 Eine Verbindung der Formel (II) wird mit einer Verbindung der Formel (III-b) umgesetzt, um eine Verbindung der Formel (I) herzustellen:
1) die Verbindung der Formel (II) wird mit R₃Cl oder R₃Br umgesetzt; die Temperatur liegt im Bereich von 0 °C-100 °C, die Reaktion wird durch anorganisches Alkali oder organisches Alkali katalysiert, wobei das anorganische Alkali NaOH, KOH, CsOH, Ba(OH)₂, Mg(OH)₂, Ca(OH), KHCO₃, K₂CO₃, Na₂CO₃ oder Cs₂CO₃ ist, das organische Alkali Natriumalkoxid, NEt₃, N(C₄H₉)₃ oder N(C₃H₇)₃ ist, die Mischung wurde 2-24 Stunden bei 0-100 °C gerührt, um eine Verbindung der Formel (I) zu ergeben; das Lösungsmittel ist ausgewählt aus der Gruppe umfassend Methanol, Ethanol, Isopropanol, C₄H₉OH, iso-C₄H₉OH, t-C₄H₉OH, C₅H₁₁OH, iso-C₅H₁₁OH, und die Mischung aus dem vorstehenden Alkohol und Wasser, wobei Alkohol:Wasser = 5:9,5-9,5:0,5 (Vol./Vol.), oder aus der Gruppe umfassend DMF, CH₂Cl₂, DMSO, THF, Dioxan, Pyrrolidonderivate, Aceton und CH₃OCH₂CH₂OCH₃;
2) die Verbindung der Formel (II) wird mit R₃COCl, (R₃CO)₂O oder R₃(CO)₂O umgesetzt; die Reaktion wird in Gegenwart von anorganischem Alkali oder organischem Alkali bei 0-100 °C durchgeführt, wobei das anorganische Alkali NaOH, KOH, CsOH, Ba(OH)₂, Mg(OH)₂, Ca(OH)₂, KHCO₃, K₂CO₃, Na₂CO₃ oder Cs₂CO₃ ist, das organische Alkali Pyridin, NEt₃, N(C₄H₉)₃, N(C₃H₇)₃ ist; die Mischung wird 2-8 Stunden bei 0-100 °C gerührt, um eine Verbindung der Formel (I) zu ergeben; das Lösungsmittel ist ausgewählt aus Pyridin, DMF, CH₂Cl₂, DMSO, THF, Dioxan und Pyrrolidonderivaten; der Katalysator DMAP wird gemäß den Reaktionsbedingungen zugegeben;
3) wenn R₁ = H, wird eine Verbindung der Formel (I) erhalten durch Hydrieren der Verbindung der Formel (II) (die Verbindung der Formel (I) mit R₁ = CH₂Ph) in Gegenwart eines Katalysators bei 0-40 °C während 1-10 h; der Katalysator ist Raney-Ni, das Lösungsmittel ist Methanol, Ethanol, Isopropanol oder das Lösungsgemisch aus dem vorstehenden Alkohol und Wasser;
(2) Herstellung aus Formel (IV): 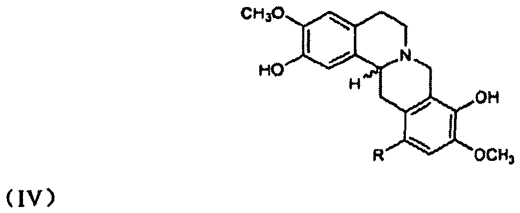 Eine Verbindung der Formel (IV) wird mit einer Verbindung der Formel (III-a) oder (III-b) umgesetzt, um eine Verbindung der Formel (I) herzustellen:
R₁Y (III-a ) oder R₃Y (III-b)
1) eine Verbindung der Formel (IV) wird mit R₁Cl oder R₁Br in unterschiedlichem Mengenverhältnis umgesetzt, um eine Verbindung der Formel (I) zu ergeben; 2) eine Verbindung der Formel (IV) wird mit R₁COCl, (R₁CO)₂O oder (R₃CO)₂O in unterschiedlichem Mengenverhältnis umgesetzt, um eine Verbindung der Formel (I) zu ergeben;
(3) Herstellung aus Formel (V): 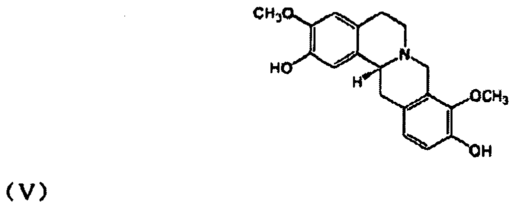 Die Verbindung der Formel (V) ist *I*-SPD. Ausgehend von *I*-SPD wird die Verbindung der Formel (I) durch Veresterung, Veretherung, Kupplung mit Aminosäure oder Demethylierung bereitgestellt;
(4) Herstellung aus Formel (VI): 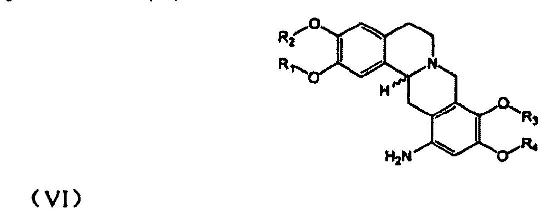 Eine Verbindung der Formel (VI) wird mit HNO₂ behandelt, um ein Diazosalz zu ergeben, welches mit einem entsprechenden Reagenz umgesetzt wird, um die Verbindung der Formel (I) zu ergeben,
wobei die Definitionen für R, R₁, R₂, R₃ und R₄ die gleichen wie in Anspruch 1 beschrieben sind.

5. Herstellungsverfahren nach Anspruch 4, wobei die Benzyloxyderivate einer Verbindung der Formel (I) in Gegenwart von Raney-Ni bei 0-40 °C während 1-10 Stunden debenzyliert werden, um eine Verbindung der Formel (I) zu ergeben; wobei das Lösungsmittel Alkohol oder das Lösungsgemisch aus Alkohol und Wasser ist.

6. Verwendung der Verbindung nach Anspruch 1 bei der Herstellung von Arzneimitteln für die Verhütung oder Behandlung einer Erkrankung des Zentralnervensystems.

7. Verwendung nach Anspruch 6, wobei es sich bei der Erkrankung des Zentralnervensystems um Schizophrenie, Parkinsonismus, eine Hyperaktivitätsstörung oder Migräne handelt.

## Revendications

1. Composés de formule (I), leurs stéréoisomères, sels pharmacologiquement acceptables et solvates : 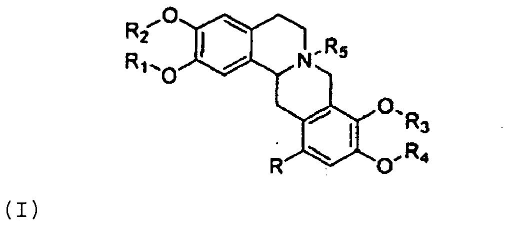 dans lesquels
R est H ;
R₁ est H, COR₇ ou R₁ et R₂ ensemble forment CH₂ ; R₇ est choisi parmi les groupes alkyle en C₁∼C₁₂, COR₈, alcoxy, ou alkyle substitué par (CH₂CH₂O)ₙR₆ (n=1∼3) ; R₆ est H, un groupe alkyle en C₁~C₃ ou alkyle substitué par un aryle ; R₈ est un groupe alcoxy ;
R₂ est H, un groupe méthyle, ou R₂ et R₁ ensemble forment CH₂ ;
R₃ est H, un groupe méthyle ou COR₇ ;
R₄ est H, un groupe méthyle ou COR₇ ;
R₅ est H, O, un groupe alkyle en C₁∼C₃, alkyle en C₁∼C₃ substitué, ou aryle, ou R₅ n'existe pas ;
Parmi les composés ci-dessus,
quand R₁=H et R₂=R₃=CH₃, R₄ est COR₇ ;
quand R₁=H et R₂=R₄=CH₃, R₃ est COR₇ ;
quand R₁ et R₂ ensemble forment CH₂, R₃ et R₄ ne peuvent pas être choisis parmi H, CH₃, ou COCH₃ en même temps ;
au moins un des R₁, R₃ ou R₄ est un groupe acyle ;
lesdites conditions excluent les composés connus suivants :
(1) 3,9,10-triméthoxy-2-acétoxy-5,8,13,13a-tétrahydro-6*H*-dibenzo[a,g]quinolizine;
(2) 2,3-méthylènedioxy-9-*p*-méthoxybenzoyloxy-10-méthoxy-5,8,13,13a-tétrahydro-6*H*-dibenzo[a,g]-quinolizine;
(3) 2,3-méthylènedioxy-9-(3,4,5-triméthoxy-benzoyloxy)-10-méthoxy-5,8,13,13a-tétrahydro-6*H-*dibenzo [a,g] quinolizine.

2. Composés selon la revendication 1, lesdits composés de formule (I) étant les composés suivants :
2,9-diacétoxy-3,10-diméthoxy-5,8,13,13a-tétrahydro-6*H*-dibenzo[a,g]quinolizine;
(-)-2,10-diacétoxy-3,9-diméthoxy-5,8,13,13a-tétrahydro-6*H*-dibenzo[a,g]-quinolizine;
(-)-2,10-divalyloxy-3,9-diméthoxy-5,8,13,13a-tétrahydro-6*H*-dibenzo[a,g]-quinolizine.

3. Composés selon la revendication 1, où les sels physiologiquement acceptables sont les sels des composés de formule (I) avec un acide, notamment l'acide chlorhydrique, l'acide bromhydrique, l'acide sulfurique, l'acide phosphorique, l'acide carbonique, l'acide sulfonique organique ; ou les sels des composés de formule (I) avec un alcali, en particulier les dérivés de métaux alcalins, notamment les dérivés du sodium ou du potassium.

4. Procédé de préparation des composés selon la revendication 1 :
(1) Préparation à partir de la formule (II) : 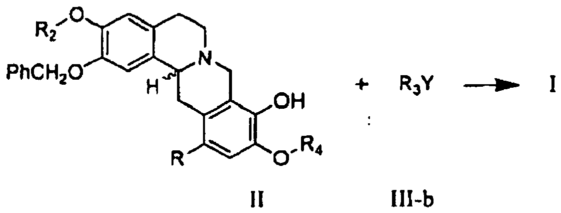 Un composé de formule (II) est mis à réagir avec un composé de formule (III-b) pour préparer un composé de formule (I) :
1) Le composé de formule (II) est mis à réagir avec R₃Cl ou R₃Br ; la température est dans la plage de 0 °C à 100 °C, la réaction est catalysée par un alcali inorganique ou un alcali organique, l'alcali inorganique étant NaOH, KOH, CsOH, Ba (OH)₂, Mg(OH)₂, Ca(OH), KHCO₃, K₂CO₃, Na₂CO₃ ou Cs₂CO₃, l'alcali organique étant un alcoolate de sodium, NEt₃, N(C₄H₉))₃ ou N(C₃H₇)_{3,} le mélange a été agité à 0-100 °C pendant 2 à 24 heures pour donner le composé de formule (I) ; le solvant est choisi dans le groupe comprenant le méthanol, l'éthanol, l'isopropanol, C₄H₉OH, iso-C₄H₉OH, t-C₄H₉OH, C₅H₁₁OH, iso-C₅H₁₁OH, et le mélange d'un alcool ci-dessus et d'eau, dans lequel alcool: eau=5:9,5-9,5:0,5 (V:V), ou dans le groupe comprenant le DMF, CH₂Cl₂, le DMSO, le THF, le dioxane, les dérivés de pyrrolidone, l'acétone et CH₃OCH₂CH₂OCH₃ ;
2) Le composé de formule (II) est mis à réagir avec R₃COCl, (R₃CO)₂O ou R₃(CO)₂O ; la réaction s'effectue en présence d'un alcali inorganique ou d'un alcali organique à 0-100 °C, l'alcali inorganique étant NaOH, KOH, CsOH, Ba(OH)₂, Mg(OH)₂, Ca(OH)₂, KHCO₃, K₂CO₃, Na₂CO₃ ou Cs₂CO₃, l'alcali organique étant la pyridine, NEt₃, N(C₄H₉)₃, N(C₃H₇)₃ ; le mélange est agité à 0-100 °C pendant 2 à 8 heures pour donner le composé de formule (I) ; le solvant est choisi parmi la pyridine, le DMF, CH₂Cl₂, le DMSO, le THF, le dioxane et les dérivés de pyrrolidone ; le catalyseur DMAP est ajouté selon les conditions de réaction ;
3) Quand R₁=H, le composé de formule (I) est obtenu par hydrogénation du composé de formule (II) (le composé de formule (I) avec R₁=CH₂Ph) en présence d'un catalyseur à 0-40 °C pendant 1 à 10 h ; le catalyseur est le Ni de Raney, le solvant est le méthanol, l'éthanol, l'isopropanol ou le mélange en solution d'un alcool ci-dessus et d'eau ;
(2) Préparation à partir de la formule (IV) : 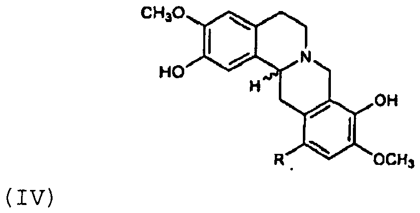 Un composé de formule (IV) est mis à réagir avec un composé de formule (III-a) ou (III-b) pour préparer un composé de formule (I) :
R₁Y (III-a) ou R₃Y (III-b)
1) Le composé de formule (IV) est mis à réagir avec R₁Cl ou R₁Br dans un rapport de quantités différent pour donner le composé de formule (I) ; 2) Le composé de formule (IV) est mis à réagir avec R₁COCl, (R₁CO)₂O ou (R₃CO)₂O dans un rapport de quantités différent pour donner le composé de formule (I) ;
(3) Préparation à partir de la formule (V) : 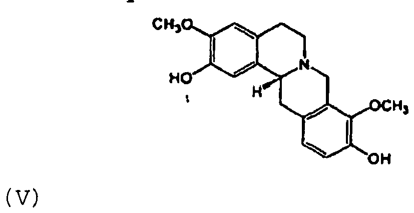 Le composé de formule (V) est la *l*-SPD. En partant de la *l*-SPD, le composé de formule (I) est obtenu par estérification, éthérification, couplage avec un acide aminé ou déméthylation ;
(4) Préparation à partir de la formule (VI) : 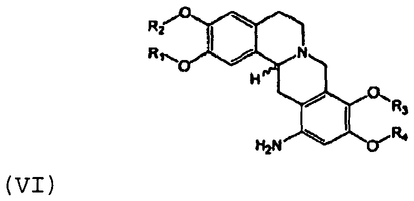 Un composé de formule (VI) est traité avec HNO₂ pour donner un sel diazo, qui est mis à réagir avec un réactif correspondant pour donner un composé de formule (I),
dans lequel les définitions de R, R₁, R₂, R₃ et R₄ sont les mêmes que celles décrites dans la revendication 1.

5. Procédé de préparation selon la revendication 4, dans lequel les dérivés benzyloxy du composé de formule (I) sont débenzylés en présence de Ni de Raney à 0-40 °C pendant 1 à 10 heures pour donner le composé de formule (I) ; le solvant est un alcool, ou le mélange en solution d'un alcool et d'eau.

6. Utilisation du composé selon la revendication 1 dans la préparation de médicaments destinés à la prévention ou au traitement d'une maladie du système nerveux central.

7. Utilisation selon la revendication 6, dans laquelle la maladie du système nerveux central est la schizophrénie, le parkinsonisme, le trouble d'hyperactivité ou la migraine.
